# EUROPEAN PATENT SPECIFICATION

(11) **EP 0 606 208 B1**
(45) Date of publication and mention of the grant of the patent: **01.05.1996**
(21) Application number: 92908264.2
(22) Date of filing: 19.03.1992
(51) Int. Cl.: A61F 13/15

(54) **A URINE INCONTINENCE PAD FOR FEMALES AND A METHOD FOR ITS PRODUCTION**
URININKONTINENZEINLAGE FÜR FRAUEN SOWIE VERFAHREN ZU IHRER HERSTELLUNG
SERVIETTE HYGIENIQUE POUR FEMMES UTILISEE EN CAS D'INCONTINENCE URINAIRE ET PROCEDE DE PRODUCTION DE LADITE SERVIETTE

(30) Priority: 02.10.1991 DK 1684/91
(43) Date of publication of application: 20.07.1994
(73) Proprietor: COLOPLAST A/S, DK-2980 Kokkedal (DK)
(72) Inventor: PEDERSEN, Jens, DK-3460 Birkeröd (DK); STRARUP, Annette, DK-3460 Birkeröd (DK)
(74) Representative: Raffnsöe, Knud Rosenstand
(86) International application number: DK9200087
(87) International publication number: WO9306804

(56) References cited:
- EP-A- 0 441 064
- WO-A-85/02110
- WO-A-86/01378
- DK-B- 153 066

## Description

The invention relates to a urinary incontinence pad for females and of the type comprising at least one relatively flat cushion-shaped absorption body made from a cellulose absorbent material arranged in a surrounding cover which at least on the side facing the user is liquid-permeable, the absorption capability at least in a part of at least one absorption body being increased by means of superabsorbing absorbent material. WO-A-86/01378 discloses a urinary incontinence pad provided with channels in the core.

Heavy demands on absorption properties and comfort in daily use are made on urinary incontinence pads which must be capable of receiving significant liquid amounts. Such an incontinence pad must inter alia be usable in combination with ordinary clothing without noticeable inconveniences, such as sounds, when the user moves. It must have a very high absorption capacity at comparatively small physical dimensions and at the same time the side facing the user must as far as possible be felt dry.

These demands are difficult to satisfy by known absorbing disposable articles consisting of a body of an absorbent material, such as cellulose fibers placed between a liquid-impermeable barrier layer and a liquid-permeable layer consisting most often of a non-woven fabric. The addition of so-called superabsorbing materials to local areas of the absorption body may entail an improvement but not always to a sufficient extent whereas the design becomes complicated because precautions must be taken to prevent the superabsorbing material from coming into contact with the skin.

In relation to the previous absorbing disposable articles it is the object of the invention to provide an improved design of the product which is in particular capable of satisfying the above mentioned functional demands on a urinary incontinence pad.

In order to accomplish this a urinary incontinence pad according to the invention is characterized in that the absorption body or bodies is/are placed between a subjacent liquid-impermeable barrier layer and a superjacent liquid-dispersive layer of a material having good liquid transfer properties in the plane of the layer, said liquid-dispersive layer being thermoplastically bonded to an absorption body containing a uniform distribution of superabsorbent material and an addition of thermolastic fibers to obtain said thermoplastic bonding of the liquid-dispersive layer and form a reinforced network structure with a view to an orderly localization of the superabsorbent material in the body, the surrounding cover consisting as a whole of a nonwoven fabric.

The use of a superjacent liquid-dispersive layer, e.g. a tissue layer on the side facing the user, and the uniform distribution of superabsorbent material which through the addition of thermoplastic material is bonded in a reinforced network structure results in a complete utilization of the absorption capacity throughout the absorption body and allow for manufacturing a high-absorbent product with comparatively small dimensions, the liquid-dispersive layer having moreover turned out to be sufficient to prevent skin contact with the superabsorbent material. The wrapping of the entire structure constituted by the absorption body, the liquid-dispersive layer and the barrier layer with a cover of nonwoven material ensures a great comfort for the user and a good retainment of the pad in ordinary undergarment, a pressure sensitive adhesive tape being applied on the rear side in a manner known per se, i.e. the side of the surrounding cover facing away from the user.

A pad appropriate for smaller liquid amounts according to the invention may simply consist of a plane non-folded cushion-like absorption body disposed in the surrounding cover.

A larger absorption capacity may be obtained in that a single absorption body with said uniform distribution of superabsorbent material in a reinforced thermoplastic network structure and the liquid-dispersive layer bonded thermoplastically thereto and the barrier layer are folded as a unitary structure at parallel folding lines into a configuration of an inverted pleat, wherein side portions of a rectangular base body constitute double-folded top layers facing the user and having a longitudinal interspace serving as a drainage channel to a subjacent coherent backing layer, the absorption body thus pleated being positioned within the surrounding cover.

Absorbing products in which the absorption body is folded in a configuration of an inverted pleat are known per se from GB patent No. 1 011 888, DK-B-117 374, US patent No. 3 592 194, FR patent No. 2 301 189 and NO patent No. 164 074.

In some of said designs, in particular intended for use as disposable napkins for children as well as for adults the purpose of the inverted-pleat-folding has mainly been to obtain an improved conformity to the user's anatomy, by providing a crutch section folded as an inverted pleat in the position of use whereas end sections beyond the crutch section are non-folded.

According to a design of the above mentioned type known from the above referenced DK-B-117 374 the inverted-pleat-folding is used to provide an accumulation space for liquid penetrated through the surrounding cover, said space being in communication with capillar interspaces between the parallel layers of absorbent material in the inverted-pleat-folding in order to obtain a high absorption capacity also in case of a sudden strong liquid flow.

According to an embodiment disclosed in NO-B-164 074 of a liquid absorbing disposable product of the above mentioned type intended for use as disposable napkins for children and adults, diapers or bandages the absorption body folded as an inverted pleat forms three successive layers, viz. the coherent backing layer and the double-folded top layer the upper side of which faces the user with an interspace forming said drainage channel with the purpose of ensuring a transfer of liquid away from the user's skin to the backing layer. In order to obtain an enhanced liquid collecting capacity in the backing layer only this layer includes an addition of superabsorbent material. In this design the surrounding cover forms in itself the liquid-impermeable barrier layer as well as the liquid-permeable layer facing the user, whereas the absorption body comprising a layer of cellulose fluff supported by a thinner layer of cellulose wad is uncovered in the surrounding cover.

The inverted-pleat-folding of the absorption body with an even ly distribution of superabsorbent material through the entire body contributes to ensuring a very high absorption capacity with small physical dimensions and by letting the liquid dispersive layer as well as the impermeable barrier layer follow the inverted-pleat-folding a good security is obtained against skin contact with the superabsorbent material and at the same time the liquid dispersive layer and the uniform distribution of the superabsorbent material ensure a good liquid distribution and utilization of the entire absorption body. Moreover, the positioning of the barrier layer between consecutive layers of the inverted-pleat-folding prevents the liquid collected in the coherent backing layer and the superjacent layer from propagating into the layer closest to user which layer is thereby still felt dry.

Finally, the thermoplastic bonding of the liquid dispersive layer to the absorbent material also in the folded design entails a fastening of the superabsorbent material in a reinforced three-dimensional network structure and secures against mutual displacement of the material layers, thereby also preventing agglomerations of absorbent material even after considerable liquid absorption.

According to a development of the invention the absorbing disposable product may comprise a number of superposed absorption bodies with associated thermoplastically bonded liquid dispersive layers, of which merely one body on the underside is connected with the barrier layer while at least one layer contains said even distribution of superabsorbent material and at least one body is a plane non-folded body.

Through variation of the quantity of superabsorbent material in the individual absorption bodies and possible incorporation of a supplementary strongly liquid transferring body between two absorption bodies it has been made possible to obtain an optimum adaptation of the absorption capacity and comfort to user's different requirements.

In an embodiment of such a disposable article with a number of absorption bodies the plane, non-folded body may thus constitute a lower body with a barrier layer on the underside, whereas an upper body is folded in a configuration of an inverted pleat with thin side portions and a central portion operating as drainage channel in contact with the liquid dispersive layer of the lower body, both bodies containing evenly distributed superabsorbent material but with a higher concentration in the lower body than in the upper body.

In a second embodiment the plane non-folded body is made from cellulose fibers without superabsorbent material and is surrounded on the underside and apart from a central portion operating as drainage channel also on the upper side by a body folded in a configuration of an inverted pleat and containing said uniform distribution of superabsorbent material, said pleated body being connected with a barrier layer on the underside below the plane body and in elevated side portions, whereas the liquid dispersive layer is thermoplastically bonded to edge portions on both sides of said drainage channel and which are lowered into the inverted-pleat-folding.

In a further embodiment such an absorbing disposable article may according to the invention comprise three superjacent plane non-folded bodies of which a lower body on the underside is connected with a barrier layer and this body as well as an upper body contain superabsorbent material, however with a larger concentration in the lower body than in the upper body, while an intermediate body of cellulose fibers and thermoplastic fibers without superabsorbent material constitutes a liquid transfer layer between the upper and the lower body.

The invention further relates to a method of manufacturing a urinary incontinence pad, said method being characterized in that a layer of liquid dispersive materials is applied to a web of absorbent material with a uniform distribution of superabsorbent material and addition of thermoplastic fibers and is by thermal treatment bonded thermoplastically to the absorbent material, following which the web possibly after superposition with one or more of subjacent webs is fastened to a web of liquid impermeable barrier material and that absorption bodies with thermoplastically bonded liquid dispersive layer and barrier layer are provided by cutting from the web configuration thereby obtained and wrapped in a nonwoven fabric web material cut off to provide the surrounding cover.

In order to provide a safe encapsulation of the absorption body with a barrier against escape of the superabsorbent material it is preferred that the severed lengths of nonwoven fabric web material are coated at the ends with an adhesive acting as barrier layer for the superabsorbent material.

The invention will now be explained in detail in the following with reference to the schematical drawings, in which
Fig. 1 is a cross-sectional view of a first embodiment of an absorbing disposable article suitable as a urinary incontinence pad according to the invention,
Fig. 2 is an enlarged section of Fig. 1,
Figs 3 and 4 are cross-sectional views of two alternative embodiments,
Figs 5, 6 and 7 are cross-sectional views of further embodiments with a number of absorption bodies, and
Fig. 8 is a plan view of an incontinence pad according to the invention.

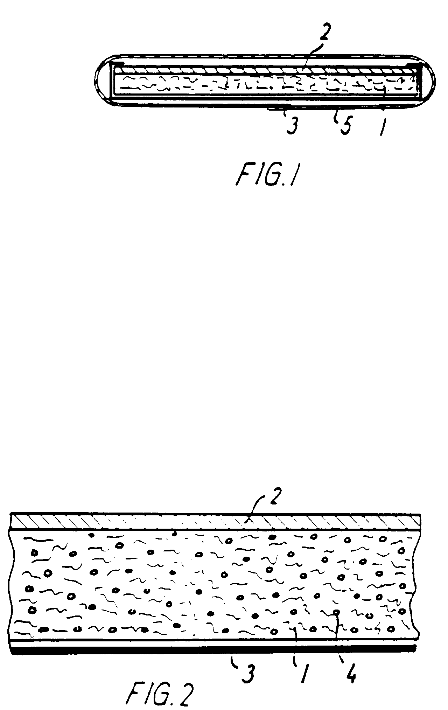

The pad illustrated in Fig. 1 has a substantially rectangular shape and consists of a comparatively flat cushion-shaped absorption body 1 placed between a liquid-dispersive layer 2 of a material with good liquid transfer properties in the plane of the layer which layer is positioned on the side which will subsequently face the user, and a liquid-impermeable barrier layer 3 on the side facing away from the user.

As a main component the absorption body 1 may consist of cellulose fibers in which superabsorbent particles, fibers or granulates 4, e.g. in an amount corresponding to 20 to 40% by weight of the absorption body, are evenly distributed throughout the body 1, as illustrated in the enlarged section in Fig. 2. According to the invention the absorption body 1 further accommodates an addition, not shown in the drawings, of thermoplastic fibers the function of which will be explained in detail in the following. The thermoplastic fibers may for instance consist of polyethylene fibers in an amount corresponding to 10 to 20% by weight of the absorption body 1.

The liquid-dispersive layer 2 on which demands are made for good liquid transfer properties in the plane of the layer may preferably consist of a tissue layer, e.g. of fibrous paper material, and covers the entire upper side of the body 1.

The liquid-impermeable barrier layer 3 is a film of an impermeable plastic material, e.g. polyethylene or polypropylene, and is so much larger than the absorption body 1 that it may bent around its edges and in over the upper side of the body so as to overlap the edge portions of the layer 2, and be connected therewith by means of an adhesive.

The pad is produced in that a web of liquid dispersive material is placed on a web of absorption material with a uniform distribution of superabsorbing particles, fibers or granules. In a following thermal treatment the thermoplastic fibers in the absorption material are softened, thereby obtaining, one on hand, a thermoplastic bonding of the liquid dispersive material to the absorption material and, on the other hand, a stabilization of the superabsorbent parricles, fibers or granulates in a reinforced three-dimensional network structure. After this treatment the absorption web is fastened to a web of liquid-impermeable barrier material with an increased width so that lateral parts of this material may be bent around the side edge of the absorption body and fastened to the liquid dispersive layer 2.

The structure consisting of the absorption body 1, the liquid-dispersive layer 2 and the barrier layer 3 is obtained by cutting convenient lengths from the webs thus united and is wrapped in a nonwoven fabric web material cut off to create the surrounding cover. In order to prevent escape of superabsorbent materials at the ends exposed by cutting and thus prevent the superabsorbent material from getting into skin contact through the nonwoven fabric web material in the surrounding cover for the absorbent material the ends 7 and 8 of the cut lengths 9 of the nonwoven fabric web material may as illustrated in the plane view in Fig. 8 be coated with an adhesive acting as barrier layer.

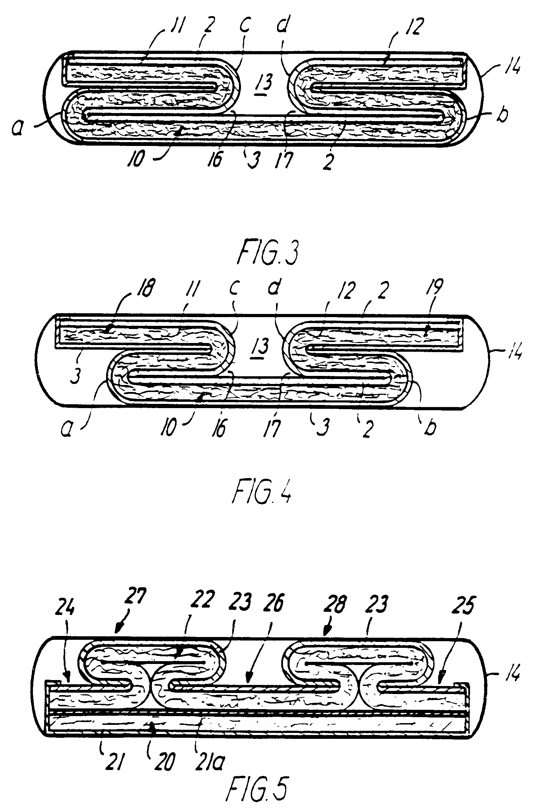

In the embodiment illustrated in Fig. 3 a base body produced in the same manner as described above is at parallel folding lines formed to an inverted pleat configuration with a coherent backing layer 10 and atop thereof two double-folded top layers 11 and 12 with a longitudinal interspace 13 operating as a drainage channel when the pad in use is placed with the top layers 11 and 12 facing the user.

In this embodiment the liquid dispersive layer 2 as well as the barrier layer 3 follow the entire configuration folded as an inverted pleat and surrounded by the cover 14.

When the pad in use receives liquid in connection with involuntary discharge of urine, the liquid hits, on one hand, the drainage channel 13 and, on the other hand, the part of the liquid dispersive layer in the top layers 11 and 12 closest to the user. Due to the good liquid transfer properties of the layer 2 in its own plane a quick dispersion of the liquid is effected. Part of the liquid flows directly through the non-woven layer of the cover 14 and the drainage channel 13 to the coherent backing layer 10 in which the dispersive effect causes an even liquid distribution also into the pockets 16 and 17 created between the backing layer 10 and the top layers 11 and 12. Even though part of the supplied liquid is also absorbed in the top layers 11 and 12 proper a larger amount will be concentrated in the backing layer 10 since, moreover, the barrier layer 3 will prevent liquid from flowing to the backing layer 10 or into the pockets 16 and 17 from seeping back to the top layers 11 and 12 so that said top layers are felt relatively dry even after use for a long time.

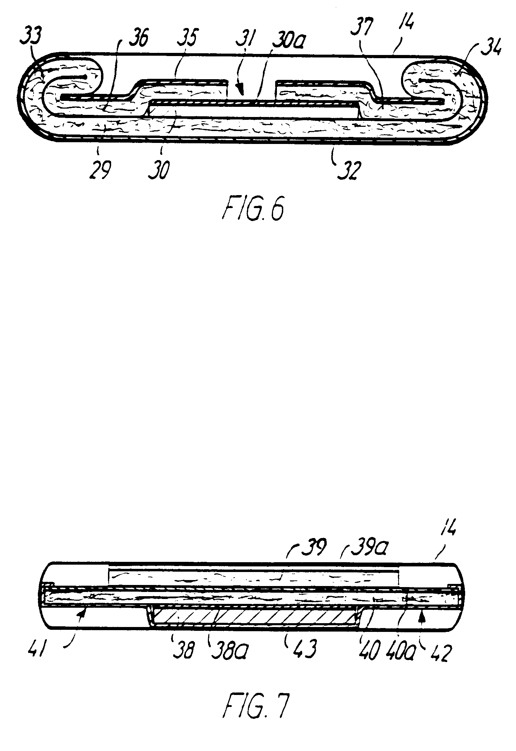

In the embodiment in Fig. 4 in which components corresponding to those illustrated in Fig. 3 have the same reference numerals, the inverted pleat folding is performed in such a way, e.g. by making the backing layer 10 narrower, that lateral parts 18 and 19 of the top layers 11 and 12 protrude beyond the side edges of the backing layer 10. An improved conformity of the comparatively compact pad to user's anatomy is thereby obtained, in the same manner as actually known from US patent No. 4 041 950.

While all of the above described embodiments comprise only a single absorption body with uniform concentration of superabsorbent material through the entire body, Figs 5, 6 and 7 schematically show embodiments with a number of absorption bodies, suitable in particular in more severe cases of incontinence, and by which an optimum fulfilling of user's specific requirements as regards absorption capacity and comfort is obtained through variation of the concentration of superabsorbent material in the individual bodies and regulation of the liquid dispersive effect and the vertical liquid transfer transversely to the absorption bodies. Thus, absorption bodies with a uniform absorbing effect across the entire surface may for instance be created, thereby avoiding spillover in case of larger amounts of flow.

In the embodiment in Fig. 5 an upper body 22 is disposed atop a plane non-folded lower body 20 which on its underside is connected with a barrier layer 21 and on the upper side has a thermoplastically bonded liquid dispersive layer 21a, said upper body being thermoplastically bonded to a superjacent liquid dispersive layer 23 and folded at parallel folding lines in a configuration of a comparatively open inverted pleat, in which thin lowered edge portions 24 and 25 and a lowered central portion 26 acting as a drainage channel are separated by two elevated portions 27 and 28.

Either of the bodies 20 and 22 contains a uniform distribution of superabsorbing cores, fibers or granulates but in a substantially larger concentration, e.g. 30 to 50% in the lower body 20 than in the upper body 22, in which the concentration may range from 10 to 20%.

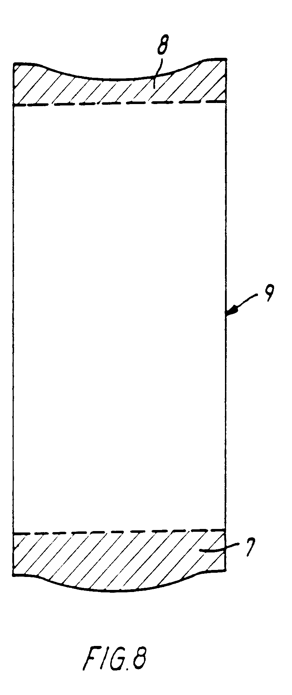

In the embodiment in Fig. 6 an absorption body 29 is folded in a configuration of an open inverted pleat about a plane, non-folded body 30 so that it covers the underside of the entire body 30 and also its upper side apart from a narrow central portion 31 acting as drainage channel.

In this embodiment only the folded body 29 contains an even distribution of superabsorbent material and an addition of thermoplastic fibers, whereas the plane non-folded body 30 consists solely of cellulose fibers (or fluff) and thermoplastic fibers and forms an inner liquid dispersive layer implying a very effective transfer to and distribution of liquid in the subjacent part of the folded body 29.

The barrier layer 32 is disposed on the underside of the folded body 29 and has such a width that it may be passed up over and cover raised side portions 33 and 34 of the body 29.

As regards the body 29 the liquid dispersive layer 35 is applied and thermoplastically bonded solely to the lowered edge portions 36 and 37 of the body 29 which are positioned on either side of the drainage channel 31.

In the embodiment in Fig. 7 the illustrated incontinence pad includes a lower body 38, an upper body 39 and an intermediary body 40 operating as an internal liquid transfer layer and having a larger width than the bodies 38 and 39 of which the lower body 38 is again a little narrower than the upper body 39. This provides for obtaining in the same manner as in the embodiment in Fig. 4 a good fit to user through the thinner edge portions 41 and 42 formed by the edge portions of the intermediate body 40 positioned outside bodies 38 and 39.

As in the embodiment in Fig. 6, the intermediate body 40 consists solely of cellulose fibers or fluff with the addition of thermoplstic fibers but without superabsorbent material, while the lower body 38 and the upper body 39 both contain an even distribution of superabsorbent material which through the addition of thermoplastic fibers is bonded in a reinforced network structure. In the same manner as in the embodiment in Fig. 6 the concentration is substantially higher in the lower body 38 than in the upper body 39.

The barrier layer 43 covers the underside of the lower body 38 and the projecting side portions 41 and 42 of the intermediate layer 40 whereas the upper sides of the bodies 38, 39 and 40 are covered by liquid dispersive layers 38a, 39a and 40a, respectively.

## Claims

1. A urinary incontinence pad for females and of the type comprising at least one relatively flat cushion-shaped absorption body (1; 6; 20, 22; 29, 30; 38, 39, 40) made from a cellulose absorbent material arranged in a surrounding cover (5, 14) of a nonwoven fabric, a liquid-impermeable barrier layer (3, 21, 32, 43) being placed in the surrounding cover (5, 14) on the side of the absorption body or bodies (1; 6; 20, 22; 29, 30; 38, 39, 40) facing away from the user and the absorption capability at least in a part of at least one absorption body being increased by means of a uniform distribution of superabsorbent material (4), the absorption body containing an addition of thermoplastic fibers to form a reinforced network structure with a view to an orderly localization of the superabsorbent material, characterized in that a liquid-dispersive layer (2; 21a, 23; 35; 38a, 39a) having good liquid transfer properties in the plane of the layer is bonded thermoplastically to said at least one absorption body (1; 6; 22; 29; 38, 39) in surface contact with the side thereof facing the user by means of said addition of thermoplastic fibers, said liquid dispersive layer (2; 21a, 23; 35; 38a, 39a) comprising a fibrous paper material acting as a barrier layer for the superabsorbent material (4) in said at least one absorption body.

2. A urinary incontinence pad according to claim 1, characterized in that a single absorption body (1) with said liquid-dispersive layer (2) bonded thermoplastically thereto and the barrier layer (3) are folded as a unitary structure (6) at parallel folding lines (a-d) into a configuration similar to an inverted pleat, wherein side portions of a rectangular base body constitute double-folded top layers (11, 12) facing the user and having a longitudinal interspace (13) serving as a drainage channel to a subjacent coherent backing layer (10), the absorption body (6) thus pleated being positioned within the surrounding cover (14).

3. A urinary incontinence pas according to claim 1 or 2, characterized in that the barrier layer (3) is folded over the side edges of the base body and covers a narrow edge portion of the liquid-dispersive layer (2) on the upper side.

4. A urinary incontinence pad according to claim 2, characterized in that the folding of the base body is effected so that lateral portions (18, 19) of the upper top layer (11, 12) facing the user project beyond the lateral edges of the coherent backing layer (10).

5. A urinary incontinence pad according to any of claims 1 to 4 characterized in that it comprises a number of superjacent absorption bodies (20, 22; 29, 30; 38, 39, 40) with associated thermoplastically bonded liquid-dispersive layers of which merely one body (20, 29, 38) on the underside is connected with the barrier layer (21, 32, 43) while at least one body contains said even distribution of the superabsorbent material and at least one body (20; 30; 38, 39, 40) is a plane non-folded body.

6. A urinary incontinence pad according to claim 5, characterized in that the the plane, non-folded body (20) is a lower body with a barrier layer (21) on the underside, whereas an upper body (22) is folded in a configuration of an inverted pleat with thin side portions (24, 25) and a central portion (26) operating as drainage channel in contact with the liquid-dispersive layer of the lower body (20), both bodies (20, 22) containing evenly distributed superabsorbent material but with a higher concentration in the lower body (20) than in the upper body (22).

7. A urinary incontinence pad according to claim 5, characterized in that the plane non-folded body (30) is made from cellulose fibers without superabsorbent material and is surrounded on the underside and apart from a central portion operating as drainage channel (31) also on the upper side by a body (29) folded in a configuration of an inverted pleat and containing said uniform distribution of superabsorbent material, said pleated body (29) being connected with a barrier layer (32) on the underside below the plane body (30) and in elevated side portions (33, 34) whereas the liquid-dispersive layer is thermoplastically bonded to edge portions (36, 37) on both sides of said drainage channel and which are lowered into the inverted-pleat-folding.

8. A urinary incontinence pad according to claim 5, characterized in that it comprises three superjacent plane non-folded bodies (38, 39, 40) of which a lower body (38) on the underside is connected with a barrier layer (43) and this body as well as an upper body (39) contain superabsorbent material, however with a larger concentration in the lower body (38) than in the upper body (39), while an intermediate body (40) of cellulose fibers and thermoplastic fibers without superabsorbent material constitutes a liquid transfer layer between the upper and the lower body (39, 38).

9. A urinary incontinence pad according to claim 8, characterized in that the intermediate body (40) has a larger width and length than the upper and the lower bodies (39, 38) and that the barrier layer (43) has a width that is so much larger than the lower body (38) that side portions of it may be folded in over and cover side portions (41, 42) of the intermediate body (40) outside lateral edges of the upper body (39).

10. A method of manufacturing a urinary incontinence pad according to any of the preceding claims, starting from a web of absorbent material containing an addition of thermoplastic fibers and a uniform distribution of superabsorbent material and comprising the steps of localizing said superabsorbent material into a reinforced net work structure in said web of absorbent mateial by a heat treatment fastening said web of absorbent material to a web of a liquid impermeable barrier material, cutting approspriate lengths from the web configuration thus obtained and wrapping said cut lengths in cut lengths of a web of non-woven fabrik material to provide a surrounding cover characterized in that a layer of a liquid dispersive material having good liquid transfer properties in the plane of the layer is applied to said web of absorbent material and is bonded thermoplastically thereto by said heat treatment.

11. A method according to claim 11, characterized in that the cut lengths of nonwoven fabric material are coated (impregnated) at the ends with an adhesive acting as barrier layer for superabsorbent material.

## Patentansprüche

1. Urininkontinenzeinlage für Frauen und von der Art, die mindestens einen verhältnismäßig flachen kissenförmigen, aus Zellstoffabsorptionsmaterial hergestellten Absorptionskörper (1; 6; 20, 22; 29, 30; 38, 39, 40) umfaßt, der in einer umschließenden Hülle (5, 14) eines nichtgewebten Textils angeordnet ist, wobei in der umschließenden Hülle (5, 14) auf der vom Benutzer abgewandten Seite des Absorptionskörpers oder der Absorptionskörper (1; 6; 20, 22; 29, 30; 38, 39, 40) eine flüssigkeitsundurchlässige Barrierenschicht (3, 21, 32, 43) vorgesehen ist, und wobei zumindest in einem Teil mindestens eines Absorptionskörpers das Absorptionsvermögen mittels einer gleichmäßigen Verteilung hochabsorbierenden Materials (4) erhöht wird, und der Absorptionskörper einen Zusatz thermoplastischer Fibern zur Bildung einer verstärkten Netzwerkstruktur mit Hinblick auf eine ordentliche Lokalisierung des hochabsorbierenden Materials umfaßt, **dadurch gekennzeichnet**, daß eine Flüssigkeitsausbreitungsschicht (2; 21a, 23; 35; 38a, 39a) mit guten flüssigkeitsleitenden Eigenschaften auf der Ebene der Schicht mittels erwähnten Zusatzes thermoplastischer Fibern in Oberflächenkontakt mit der dem Benutzer zugewandten Seite an erwähnten mindestens einem Absorptionskörper (1; 6; 22; 29; 38, 39) thermoplastisch gebunden ist, wobei erwähnte Flüssigkeitsausbreitungsschicht (2; 21a, 23; 35; 38a, 39a) ein faseriges Papiermaterial umfaßt, das als eine Barrierenschicht für das hochabsorbierende Material (4) in erwähntem mindestens einem Absorptionskörper dient.

2. Urininkontinenzeinlage nach Anspruch 1, **dadurch gekennzeichnet**, daß ein einzelner Absorptionskörper (1) mit erwähnter an diesen thermoplastisch gebundener Flüssigkeitsausbreitungsschicht (2) und die Barrierenschicht (3) als eine Gesamtstruktur (6) entlang parallelen Faltenlinien (a-d) in einer umgekehrten Falten ähnlichen Konfiguration gefaltet sind, in der Seitenpartien eines rechteckigen Basiskörpers zweifach gefaltete, dem Benutzer zugewandte Oberschichten (11, 12) mit einem längs verlaufenden Zwischenraum (13) bilden, der als ein Dränkanal zu einer darunterliegenden zusammenhängenden Hinterschicht (10) dient, wobei der derart gefaltete Absorptionskörper (6) in der umschließenden Hülle (14) angeordnet ist.

3. Urininkontinenzeinlage nach Anspruch 1 oder 2, **dadurch gekennzeichnet**, daß die Barrierenschicht (3) über die Seitenkanten des Basiskörpers gefaltet ist und eine schmale Kantenpartie der Flüssigkeitsausbreitungsschicht (2) auf der Oberseite deckt.

4. Urininkontinenzeinlage nach Anspruch 2, **dadurch gekennzeichnet**, daß das Falten des Basiskörpers derart ausgeführt wird, daß Seitenteile (18, 19) der oberen dem Benutzer zugewandten Oberschicht (11, 12) über die Seitenkanten der zusammenhängenden Hinterschicht (10) hinausragen.

5. Urininkontinenzeinlage nach einem der Ansprüche 1 bis 4, **dadurch gekennzeichnet**, daß sie eine Anzahl übereinanderliegender Absorptionskörper (20, 22; 29, 30; 38, 39, 40) mit dazugehörigen thermoplastistisch gebundenen Flüssigkeitsausbreitungsschichten umfaßt; von welchen nur ein Körper (20, 29, 38) auf der Unterseite mit der Barrierenschicht (21, 32, 43) verbunden ist, während mindestens ein Körper erwähnte gleichmäßige Verteilung des hochabsorbierenden Materials aufweist, und mindestens ein Körper (20; 30; 38, 39, 40) ein flacher, nichtgefaltener Körper ist.

6. Urininkontinenzeinlage nach Anspruch 5, **dadurch gekennzeichnet**, daß der flache, nichtgefaltene Körper (20) ein unterer Körper mit einer Barrierenschicht (21) auf der Unterseite ist, während ein oberer Körper (22) in eine Konfiguration einer umgekehrten Faltung mit dünnen Seitenpartien (24, 25) und einer zentralen Partie (26) gefaltet ist, die als Dränkanal in Kontakt mit der Flüssigkeitsausbreitungsschicht des unteren Körpers (20) dient, wobei beide Körper (20, 22) gleichmäßig verteiltes hochabsorbierendes Material, aber mit einer höheren Konzentration in dem unteren Körper (20) als in dem oberen Körper (22) aufweisen.

7. Urininkontinenzeinlage nach Anspruch 5, **dadurch gekennzeichnet**, daß der flache, nichtgefaltene Körper (30) aus Zellstoffibern ohne hochabsorbierendes Material hergestellt ist, und auf der Unterseite und, abgesehen von einem zentralen Teil, der als Dränkanal (31) dient, auch auf der oberen Seite von einem Körper (29) umschloßen ist, der in einer Konfiguration einer umgekehrten Faltung gefaltet ist und die gleichmäßige Verteilung von hochabsorbierendem Material aufweist, wobei erwähnter plissierter Körper (29) auf der Unterseite unter dem flachen Körper (30) und in erhöhten Seitenpartien (33, 34) mit einer Barrierenschicht (32) verbunden ist, während die Flüssigkeitsausbreitungsschicht an Kantenpartien (36, 37) auf beiden Seiten erwähntes Dränkanals thermoplastisch gebunden ist, welche Kantenpartien in die umgekehrte Faltung gesenkt werden.

8. Urininkontinenzeinlage nach Anspruch 5, **dadurch gekennzeichnet**, daß sie drei übereinanderliegende, flache, nichtgefaltene Körper (38, 39, 40) umfaßt, von welchen ein unterer Körper (38) auf der Unterseite mit einer Barrierenschicht (43) verbunden ist, und daß dieser Körper sowie ein oberer Körper (39) hochabsorbierendes Material beinhalten, aber mit einer größeren Konzentration im unteren Körper (38) als im oberen Körper (39), während ein Zwischenkörper (40) aus Zellstoffibern und thermoplastischen Fibern ohne hochabsorbierendes Material eine flüssigkeitsleitende Schicht zwischen dem oberen und dem unteren Körper (39, 38) bildet.

9. Urininkontinenzeinlage nach Anspruch 8, **dadurch gekennzeichnet**, daß der Zwischenkörper (40) eine größere Breite und Länge als die der oberen und unteren Körper (39, 38) aufweist, und daß die Barrierenschicht (43) eine Breite aufweist, die um so viel größer als die Breite des unteren Körpers (38) ist, daß die Seitenpartien der Barrierenschicht über Seitenpartien (41, 42) des Zwischenkörpers (40) außerhalb der Seitenkanten des oberen Körpers (39) gefalten werden können und diese decken.

10. Verfahren zur Herstellung einer Urininkontinenzeinlage nach einem der vorhergehenden Ansprüche, ausgehend von einem Gewebe von absorbierendem Material mit einem Zusatz thermoplastischer Fibern und einer gleichmäßigen Verteilung superabsorbierenden Materials, und umfaßend die Stufen von Lokalisierung erwähnten superabsorbierenden Materials in eine verstärkte Netzwerkstruktur in erwähntem Gewebe von absorbierendem Material durch eine Wärmebehandlung, die erwähntes Gewebe von absorbierendem Material an ein Gewebe von flüssigkeitsundurchlässigem Barrierenmaterial befestigt, passende Längen aus der dadurch erhaltenen Gewebekonfiguration abschneidet, und erwähnte abgeschnittene Längen in Schnittlängen eines Gewebes von nichtgewebtem Textilmaterial zur Bildung einer umschließenden Hülle umwickelt, **dadurch gekennzeichnet**, daß eine Schicht eines flüssigkeitsausbreitenden Materials mit guten flüssigkeitsleitenden Eigenschaften in der Ebene der Schicht auf erwähntes Gewebe von absorbierendem Material gelegt und durch erwähnte Wärmebehandlung an dieses thermoplastisch gebunden wird.

11. Verfahren nach Anspruch 10, **dadurch gekennzeichnet**, daß die abgeschnittenen Längen von nichtgewebten Textilmaterial an den Enden mit einem als Barrierenschicht für hochabsorbierendes Material dienenden Klebemittel belegt (imprägniert) werden.

## Revendications

1. Serviette hygiénique pour incontinence urinaire féminine et du type comprenant au moins un corps absorbant, relativement plat, en forme de coussinet (1; 6; 20, 22; 29, 30; 38, 39, 40) à base d'un matériau cellulosique absorbant disposé dans une enveloppe (5, 14) en non-tissé, une couche barrière imperméable aux liquides (3, 21, 32, 43) étant placée dans l'enveloppe (5, 14) du coté du ou des corps absorbants (1; 6; 20, 22; 29, 30; 38, 39, 40) qui ne fait pas face à l'utilisateur, la capacité d'absorbtion dans au moins une partie d'au moins un corps absorbant étant accrue à l'aide d'une répartition uniforme d'un matériau superabsorbant (4), le corps absorbant comportant un supplément de fibres thermoplastiques pour former une structure en réseau renforcé dans le but d'une localisation ordonnée du matériau superabsorbant, caractérisée par le fait qu'une couche dispersive de liquides (2; 21a, 23; 35; 38a, 39a), présentant de bonnes propriétés de transfert de liquide, est thermoplastiquement liée audit au moins un corps absorbant (1; 6; 22; 29; 38, 39) en contact de surface avec le coté de celui-ci faisant face à l'utilisateur, à l'aide dudit supplément de fibres thermoplastiques, ladite couche dispersive de liquides (2; 21a, 23; 35; 38a, 39a) comportant un matériau en fibres de papier agissant en tant que couche barrière pour le matériau superabsorbant (4) dans ledit au moins un corps absorbant.

2. Serviette hygiénique pour incontinence urinaire selon la revendication 1, caractérisée par le fait qu'un corps absorbant unique (1) avec ladite couche dispersive de liquide (2) qui lui est thermoplastiquement liée et la couche barrière (3) sont repliés en une structure unitaire (6) le long de lignes parallèles de pliage (a-d) en une configuration semblable à des plis inversés, les portions latérales d'un corps de base rectangulaire formant des couches supérieures à double pli (11, 12) faisant face à l'utilisateur et comportant un espace intermédiaire longitudinal (13) servant de canal de drainage vers une garniture cohérente, sous-jacente (10), le corps absorbant (6) ainsi plissé étant logé dans l'enveloppe (14) qui l'entoure.

3. Serviette hygiénique pour incontinence urinaire selon la revendication 1 ou 2, caractérisée par le fait que la couche barrière (3) est repliée sur les bords latéraux du corps de base et recouvrent une étroite portion de bord de la couche dispersive de liquides (2) sur la face supérieure.

4. Serviette hygiénique pour incontinence urinaire selon la revendication 2, caractérisée par le fait que le pliage du corps de base est effectué de telle manière que les portions latérales (18, 19) de la couche supérieure (11, 12) faisant face à l'utilisateur s'étendent au-delà des bords latéraux de la couche de garniture cohérente (10).

5. Serviette hygiénique pour incontinence urinaire selon l'une quelconque des revendications 1 à 4, caractérisée par le fait qu'elle comporte un nombre de corps absorbants superposés (20, 22; 29, 30; 38, 39, 40) avec couches thermoplastiquement associées, dispersives de liquides, et dont un seul corps (20, 29, 38) sur sa face inférieure est relié à la couche barrière (21, 32, 43), tandis qu'au moins un corps contient une répartition uniforme du matériau superabsorbant et au moins un corps (20; 30; 38, 39 40) est un corps plan, non plié.

6. Serviette hygiénique pour incontinence urinaire selon la revendication 5, caractérisée par le fait que le corps plan, non plié (20) est un corps inférieur avec une couche barrière (21) sur la face inférieure, un corps supérieur (22) étant plié en une configuration à plis inversés à minces bords latéraux (24, 25) et une portion centrale (26) formant canal de drainage en contact avec la couche dispersive de liquides du corps inférieur (20), les deux corps (20, 22) contenant du matériau superabsorbant uniformément distribué mais à concentration plus élevée dans le corps inférieur (20) que dans le corps supérieur (22).

7. Serviette hygiénique pour incontinence urinaire selon la revendication 5, caractérisée par le fait que le corps plan, non plié (20) est constitué de fibres de cellulose sans matériau superabsorbant et est entouré sur le dessous et, hormis une portion centrale formant canal de drainage (31), aussi sur le dessus par un corps (29) plié en une configuration à plis inversés et contenant ladite répartition uniforme de matériau superabsorbant, ledit corps plié (29) étant relié à une couche barrière (32) sur la face inférieure du corps plan (30) et dans les portions latérales surélevées (33, 34), la couche dispersive de liquides étant thermoplastiquement liée aux portions latérales (36, 37) situées de part et d'autre dudit canal de drainage et rabaissées dans les replis inversés.

8. Serviette hygiénique pour incontinence urinaire selon la revendication 5, caractérisée par le fait qu'elle comprend trois corps plans, superposés, non pliés (38, 39, 40), dont un corps inférieur (38), par sa face inférieure, est lié à une couche barrière (43), ce corps ainsi qu'un corps supérieur (39) contenant du matériau superabsorbant, avec, cependant, une concentration plus importante dans le corps inférieur (38) que dans le corps supérieur (39), un corps intermédiaire (40) en fibres de cellulose et fibres thermoplastiques sans matériau superabsorbant constituant une couche de transfert de liquides entre le corps supérieur (39) et le corps inférieur (38).

9. Serviette hygiénique pour incontinence urinaire selon la revendication 8, caractérisée par le fait que le corps intermédiaire (40) présente une largeur et une longueur plus importantes que le corps supérieur (39) et le corps inférieur (38) et que la couche barrière (43) a une largeur suffisamment plus grande que celle du corps inférieur (39) pour qu'elle puisse être repliée autour et recouvrir les portions latérales (41, 42) du corps intermédiaire (40), au-delà des bords latéraux du corps supérieur (39).

10. Procédé de fabrication d'une serviette hygiénique pour incontinence urinaire selon l'une quelconque des revendications précédentes, à partir d'une bande de matériau absorbant contenant un supplément de fibres thermoplastiques et une répartition uniforme de matériau superabsorbant, comprenant les étapes de localisation dudit matériau superabsorbant en une structure en réseau renforcé dans ladite bande de matériau absorbant, par traitement thermique liant ladite bande de matériau absorbant à une bande de matériau barrière imperméable aux liquides, découpe de la configuration en bande ainsi obtenue en longueurs appropriées et enveloppement desdites longueurs coupée dans des longueurs coupées d'une bande de matériau non tissé pour former une enveloppe de recouvrement, caractérisé par le fait qu'une couche de matériau dispersif de liquides présentant de bonnes propriétés de transfert de liquides dans le plan de la couche est appliquée sur ladite bande de matériau absorbant et est thermoplastiquement liée à cette bande par ledit traitement thermique.

11. Procédé selon la revendication 10, caractérisé par le fait que les longueurs coupées de matériau non tissé sont revêtues (imprégnées) aux extrémités avec un adhésif agissant comme couche barrière pour le matériau superabsorbant.
